(19) Europäisches Patentamt
European Patent Office
Office européen des brevets

(11) Veröffentlichungsnummer: **0 263 493**
**A2**

# (12) EUROPÄISCHE PATENTANMELDUNG

(21) Anmeldenummer: **87114589.2**

(51) Int. Cl.4: **A61K 31/19**, A61K 31/575

(22) Anmeldetag: **06.10.87**

Patentansprüche für folgenden Vertragsstaaten: GR, AT und ES

(30) Priorität: **07.10.86 US 916150**

(43) Veröffentlichungstag der Anmeldung: **13.04.88 Patentblatt 88/15**

(84) Benannte Vertragsstaaten: **AT BE CH DE ES FR GB GR IT LI LU NL SE**

(71) Anmelder: **F. HOFFMANN-LA ROCHE & CO. Aktiengesellschaft**

**CH-4002 Basel(CH)**

(72) Erfinder: **Behl, Charanjit Rai**
**40 Franklin Avenue**
**Nutley, N.J. 07110(US)**
Erfinder: **Unowsky, Joel**
**37 Fellswood Drive**
**Livingston, N.J. 07039(US)**

(74) Vertreter: **Lederer, Franz, Dr. et al**
**Patentanwält Dr. Franz Lederer Lucile**
**Grahnstrasse 22**
**D-8000 München 80(DE)**

## (54) Pharmazeutische Präparate zur verbesserten gastrointestinalen Resorption.

(57) Oral verabreichbares pharmazeutisches Präparat, enthaltend (a) eine therapeutisch wirksame Substanz, deren Bioverfügbarkeit in Gegenwart eines Resorptionsverstärkers verbessert wird, eine wirksame Menge von (b) (i) Chenodeoxycholsäure, Deoxycholsäure oder pharmazeutisch verwendbare Salze davon, gegebenenfalls in Kombination mit (ii) einem Synergisten für die Komponente (b) (i), und (c) einen pharmazeutisch anwendbaren Träger für diese Inhaltsstoffe.

EP 0 263 493 A2

## Pharmazeutische Präparate zur verbesserten gastrointestinalen Resorption

Die Erfindung betrifft oral verabreichbare pharmazeutische Präparate, die Chenodeoxycholsäure oder Deoxycholsäure oder ein Salz dieser Säuren allein oder in Kombination mit einem Synergisten. z.B. Glyceriden, enthalten und die die Resorption therapeutischer Substanzen im Gastrointestinaltrakt verstärken.

Generell kann die Verabreichung therapeutischer Substanzen auf verschiedenen Wegen bewerkstelligt werden, wobei die hauptsächlichen die orale, rektale und parenterale Verabreichung sind. Im Idealfall sollte ein Heilmittel in jeder dieser Formen verabreichbar sein. In der Praxis werden jedoch viele Heilmittel einschliesslicher einer Anzahl Antibiotika nicht ohne weiteres oder hinreichend im Gastrointestinaltrakt resorbiert, was für die meisten Zwecke ihre Verwendbarkeit in oralen Dosierungsformen erheblich beschränkt oder ausschliesst. Bei einigen therapeutischen Substanzen, wie schwefelhaltigen Heilmitteln, die zur Behandlung von Schleimhautenzündungen verwendet werden, kann mangelhafte Resorptionsfähigkeit willkommen sein, da sie die Zeitdauer der Einwirkung des Heilmittels auf das entzündete Gewabe verlängert. Bei den meisten Antibiotika wie auch bei anderen Heilmitteln ist die Resorption durch den Magen oder den Intestinaltrakt eine Notwendigkeit und die Verabreichung als Tablette, Pille oder Kapsel ist einfacher und bringt in den meisten Fällen physiologische Vorteile mit sich.

Ein Aspekt der vorliegenden Erfindung betrifft ein oral verabreichbares pharmazeutisches Präparat, enthaltend (a) eine therapeutisch wirksame Substanz, deren Bioverfügbarkeit in Gegenwart eines Resorptionsverstärkers verbessert wird, eine wirksame Menge von (b) (i) Chenodeoxycholsäure, Deoxycholsäure oder pharmazeutisch verwendbare Salze davon, gegebenenfalls in Kombination mit (ii) einem Synergisten für die Komponente (b) (i), und (c) einen pharmazeutisch anwendbaren Träger für diese Inhaltsstoffe.

Synergisten für die Komponente (b)(i), die als Komponente (b)(ii) in den erfindungsgemässen Präparaten enthalten sein können, sind im allgemeinen Fettsäureester, wie Glycerin-und Sucroseester. Vorzugsweise ist die Komponente (b)(ii) ein Ester einer Fettsäure mittlerer Kettenlänge, beispielsweise einer $C_8$-$C_{12}$-Fettsäure. Besonders bevorzugt sind Mono-, Di-und Triester, beispielsweise Glyceride von Fettsäuren mittlerer Kettenlänge.

Ein anderer Aspekt der Erfindung betrifft die Verwendung von (i) Chenodeoxycholsäure, Deoxycholsäure, oder pharmazeutisch verwendbaren Salzen davon, gegebenenfalls in Kombination mit (ii) einem Synergisten für die Komponente (i) als Resorptionsverstärker zur Herstellung von oral verabreichbaren pharmazeutischen Präparaten.

Wie im folgenden weiter ausgeführt werden wird, ist die Erfindung auf einen breiten Bereich therapeutisch wirksamer Substanzen anwendbar, die normalerweise in Folge schlechter gastrointestinaler Resorption nicht oral verabreicht werden können, und auch auf therapeutische Substanzen, die normalerweise oral verabreichbar werden können aber deren orale Wirksamkeit verbessert werden soll. Insbesondere ist hier die Familie oder Klasse der $\beta$-Lactam-Antibiotika zu erwähnen, von denen eine Anzahl in den Beispielen angewandt wird, um die Durchführung der Erfindung zu illustrieren, und Insulin.

Die erfindungsgemässen Präparate können als feste, halbfeste oder flüssige orale Dosierungsformen hergestellt werden, wie Pillen, Tabletten, Kapseln, Pulver und Granulate unter Verwendung von konventionellen Technologien.

Die Präparate können auch für frühzeitige oder verzögerte Freisetzung im Gastrointestinaltrakt formuliert werden.

Die erwähnten Resorptionsverstärker sind erfindungsgemäss für einen weiten Bereich therapeutischer Substanzen vorgesehen, im allgemeinen für jede therapeutische Substanz, die bei oraler Anwendung durch Anwendung dieser Resorptionsverstärker aktiver wird. Die für die vorliegende Erfindung verwendbaren therapeutischen Substanzen können beispielsweise Retinoide, Peptide, Polypeptide, ernährungswichtige Mineralien (beispielsweise Eisen-, Calcium-, Kalium-und Zinksalze) Proteine (beispielsweise Insulin), Antibiotika (insbesondere $\beta$-Lactame) und Vitamine sein.

Bevorzugte therapeutische Substanzen für die erfindungsgemässen Präparate sind $\beta$-Lactam-Antibiotika, d.h. Verbindungen mit dem $\beta$-Lactamring

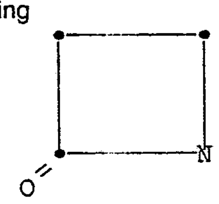

der an verschiedenen Stellen des Ringes substituiert sein kan und/oder mit anderen Ringsystemen

verbunden sein kann, die für sich substituiert oder unsubstituiert sein können. Beispiele bekannter β-Lactam-Antibiotika sind Penicilline, Cephalosporine und monocyclische β-Lactame.

Besonders bevorzugte β-Lactam-Antibiotika sind Verbindungen der Formel

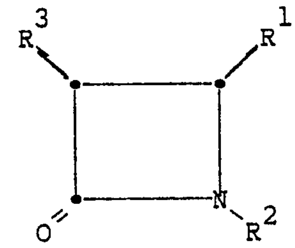

worin $R^1$ Wasserstoff, Alkyl oder substituiertes Alkyl, $R^2$ $SO_3^-$ $M^+$, $M^+$ ein Proton oder Kation, $R^3$ eine Acylaminogruppe oder Hydroxyalkyl oder $R^1$ und $R^2$ zusammen mit dem β-Lactam (Azetidinon)-Ring eine Struktur der Formel

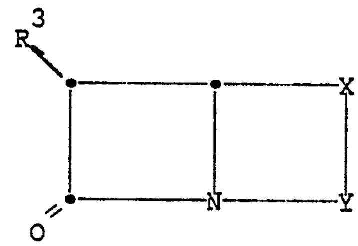

bilden, worin X S, O, SO, $SO_2$ oder $CH_2$ und Y eine Gruppe

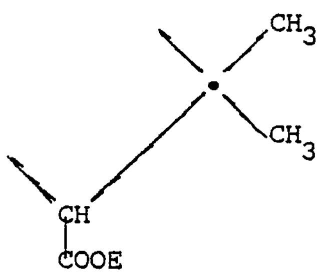

oder

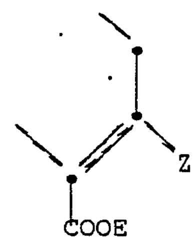

darstellen,
wobei das Kohlenstoffatom, das die -COOE-Gruppe trägt, an das Stickstoffatom des β-Lactamringes gebunden ist, Z Wasserstoff, Halogen, Alkoxy oder $CH_2$-T darstellt, T Wasserstoff, Alkyl -CO-O-, Pyridinium Carboxamidopyridinium, Aminopyridinium, Carbamoyloxy, Azido, Cyano, Hydroxyl, die Gruppe -S-Phenyl, die auch substituiert sein kann, oder die Gruppe -S-Het darstellt, wobei Het ein gegebenenfalls substituiert 5-oder 6-gliedriger heterocyclischer Ring ist und E Wasserstoff, eine pharmazeutisch anwendbare Estergruppe oder ein salzbildendes Kation darstellt.

Besonders bevorzugte β-Lactam-Antibiotika und deren pharmazeutisch anwendbare Salze, Ester und Hydrate sind Ceftriaxon, ein beispielsweise im U.S.-Patent Nr. 4,327,210 beschriebenes Cephalosporin; Carumonam, ein monocyclisches β-Lactam, das beispielsweise in der europäischen Patentschrift Nr. EP 73063 beschrieben ist; Piperacillin, ein beispielsweise im U.S.-Patent Nr. 4,122,090 beschriebenes Penicillin; Cefamandol, ein beispielsweise im U.S.-Patent Nr. 3,641,021 beschriebenes Cephalosporin; Mezlocillin, ein beispielsweise im U.S.-Patent Nr. 3,974,142 beschriebenes Penicillin; Cefazolin, ein beispielsweise im U.S.-Patent Nr. 3,516,997 beschriebenes Cephalosporin. Weitere Beispiele sind Cefoxitin, Amdinocillin, Moxalactam, Aztreonam, Cefotaxim, Cefmenoxin, Ampicillin, Cefoperazon, Cefsulodin und Thienamycin.

Wie erwähnt, werden als Resorptionsverstärker erfindungsgemäss Chenodeoxycholsäure oder Deoxycholsäure oder vorzugsweise ein pharmazeutisch anwendbares Salz einer diesen Säuren verwendet. Die Salze können durch Umsetzung der Säure mit einer Base, die ein nicht-toxisches, pharmakologisch und pharmazeutisch anwendbares Kation enthält, erhalten werden. Im allgemeinen kommt jede Base, die ein

Salz mit einer Carbonsäure bildet und deren pharmakologischen Eigenschaften keine erwünschten physiologischen Effekte bei Warmblütern hervorruft, erfindungsgemäss in Betracht. Geeignete Basen sind beispielsweise Alkalimetall-und Erdalkalimetallhydroxide und -carbonate, wie Natriumhydroxid, Kaliumhydroxid, Calciumhydroxid, Kaliumcarbonat, Ammoniak, primäre sekundäre und tertiäre Amine, wie Monoalkylamine, Dialkylamine, Trialkylamine; stickstoffhaltige heterocyclische Amine, wie Piperidin, und basische Aminosäuren wie Lysin. Die so hergestellten pharmazeutisch anwendbaren Salze sind funktionelle Aequivalent der entsprechenden Chenodeoxycholsäure und Deoxycholsäure und der Auswahlbereich der Salze wird erfindungsgemäss nur durch das Kriterium begrenzt, dass die bei der Bildung der Salze angewandten Basen sowhol nicht-toxisch als auch physiologisch und pharmazeutisch anwendbar sind.

Vorzugsweise, jedoch nicht notwendigerweise, wird der oben erwähnte Resorptionsverstärker mit einem zweiten Stoff angewandt, der die Resorption synergistisch weiter verbessert und der vorzugsweise eine $C_8$-$C_{12}$-Fettsäure-Mono-, Di-oder Triglycerid oder ein Gemisch von zwei oder mehreren solcher Glyceride ist. Besonders bevorzugt sind Gemische mit einer grösseren Menge, d.h. mehr als 50 Gew.-% eines Monoglycerids einer $C_8$-$C_{12}$-gesättigten Fettsäure und einer kleineren Menge eines Di-und/oder Triglycerids einer $C_8$-$C_{12}$-gesättigten Fettsäure. Geeignete Materialien sind unter dem Namen "CAPMUL" von der Firma Stokely-Van Camp, Columbus, Ohio, erhältlich.

Die wirksame Menge des Resorptionsverstärkers, d.h. der Komponente (b)(i) und (b)(ii) in den erfindungsgemässen Präparaten hängt von Faktoren wie der angewandeten therapeutischen Substnaz, der Schwere der Erkrankung und dem Alter des zu behandelnden Patienten ab.

Im allgemeinen ist es für orale antibiotische Kompositionen bevorzugt, etwa 5 bis etwa 1000 mg, insbesondere 50 bis 500 mg, eines jeden Resorptionsverstärkers pro Dosiseinheit des Präparats zu verwenden. Derartige Präparate enthal ten $\beta$-Lactam-Antibiotika in Mengen von etwa 25 bis etwa 2500 mg, häufiger 100 bis 1500 mg pro Dosiseinheit.

Insulin-haltige orale Präparate enthaltend typischerweise etwa 5-100 mg, insbesondere etwa 50 bis etwa 500 mg Resorptionsverstärker, wobei das Insulin normalerweise in Mengen von 0,1 bis etwa 20'000 Einheiten, meistens von etwa 1 bis etwa 100 Einheiten pro Dosiseinheit anwesend ist.

Der Ausdruck "Dosiseinheit" wird hier im konventionellen Sinne verwendet, um eine einzelne Verabreichung des Medikaments in der oben angegebenen Menge zu bezeichnen.

Als Träger kann jeder pharmazeutisch anwendbare feste, halbfester oder flüssiger Träger, in dem die Komponenten löslich oder ohne weiteres dispergierbar sind, verwendet werden. Beispiele sind Kakaobutter, Polyäthylenglykole, Polypropylenglykole, Glycerogelatine, Methylcellulose, Carboxymethylcellulose und Suppocire* (Gattefosse Corp., Elmsford, NY). Bevorzugt für die diesen Zwecke sind Gemische von Triglyceriden von $C_{12}$-$C_{18}$ natürlichen gesättigten Fettsäuren, vorzugsweise vegetabilen Fettsäuren, die eine geradzahlige Kohlenstoffatomzahl haben ($C_{12}$, $C_{14}$, $C_{16}$. Besonders bevorzugt sind die Produkte der WITEPSOL-Linie (Dynamit Nobel) von denen einige nachstehend angeführt sind:

WITEPSOL E75 (Smp. 37-39°C, OH ZAHL 220-230)
WITEPSOL E76 (Smp. 37-39°C, OH Zahl 30-40)
WITEPSOL E79 (Smp. 36-38°C, OH Zahl 25-35)
WITEPSOL E85 (Smp. 42-44°C, OH Zahl 15 max.)
WITEPSOL H5 (Smp. 34-36°C, OH Zahl 5 max.)
WITEPSOL H12 (Smp. 32-33.5°C, OH Zahl 15 max.)
WITEPSOL H15 (Smp. 33.5-35.5°C, OH Zahl 15 max.)
WITEPSOL H19 (Smp. 33.5°C, OH Zahl 20-20)
WITEPSOL S52 (Smp. 32-33.5°C, OH Zahl 50-65)
WITEPSOL S55 (Smp. 33.5-35.5°C, OH Zahl 50-65)
WITEPSOL S58 (Smp. 32-33.5°C, OH Zahl 60-70)
WITEPSOL W25 (Smp. 33.5-35.5°C, OH Zahl 20-30)
WITEPSOL W31 (Smp. 35-37°C, OH Zahl 25-35)
WITEPSOL W35 (Smp. 33.5-35.5°C, OH Zahl 40-50)
WITEPSOL W45 (Smp. 33.5-35.5°C, OH Zahl 40-50)

Die Menge der Komponente (c) bewegt sich im Rahmen des für pharmazeutische Trägerstoffe Ueblichen, d.h. in Mengen, die einfach und sicher verabreicht werden können.

Obschon die Erfindung sehr ausführlich in bezug auf $\beta$-Lactam-Antibiotika als Wirkstoffe beschrieben wird, sei nochmals betont, dass die Erfindung auch für die Anwendung auf andere therapeutisch wirksame Substanzen konzipiert ist, seien sie natürlich vorkommend oder halbsynthetisch oder synthetisch. Beispielsweise kann die gastrointestinale Resorption von Insulin, das üblicherweise mit befriedigendem Effekt nur parenteral verabreicht werden kann, mit den erfindungsgemässen Mitteln verstärkt werden.

Die bevorzugte Methode der Verarbreichung therapeutischer Substanzen, beispielsweise von $\beta$-Lactam-Antibiotika mit einem der beiden Resorptionsverstärker ist eine Dosierungsform, die mit einem magensaftresistenten Ueberzug versehen ist, d.h. eine mit einem solchen Ueberzug versehene feste Dosierungsform. Die Trägerstoffe können entweder in fester oder flüssiger Form vorliegen und können in Hart-oder Weichgelatinekapseln abgefüllt sein, oder der flüssige Trägerstoff kann an einen geeigneten Träger adsorbiert sein, so dass ein freifliessendes Pulver vorleigt, und dann in eine Kapsel abgefüllt werden oder alternativ zu Pillen oder Tabletten verpresst werden. Andere Dosierungsformen können Verabreichungssysteme darstellen, die nicht mit einem Ueberzug versehen sind, d.h. Kapseln oder Tabletten, in denen das Antibiotikum und der Resorptionsverstärker selbst mit einem magensaftresistenten Ueberzug versehen sind, beispielsweise in Form von Mikrokapseln, mit denen eine Hart-oder Weichgelatinekapsel gefüllt ist oder die zu einer Tablette ver presst sind. Andere mögliche Dosierungsformen sind Mikrokapseln oder Beadlets, die mit einem magensaftresistenten Ueberzug versehen sind und die das Antibiotikum oder eine andere therapeutische Substanz zusammen mit dem Resorptionsverstärker enthalten und die danach in Kapseln abgefüllt sind, die wiederum mit einem magensaftresistenten Ueberzug versehen sein können.

Die Verwendung von magensaftresistenten Ueberzügen in der oben beschriebenen Weise dient dazu, dass $\beta$-Lactam-Antibiotikum oder andere entsprechend empfindliche therapeutische Substanzen vor der Magenflüssigkeit zu schützen und die therapeutische Substanz und den Resorptionsverstärker optimal in den Dünndarm zu bringen.

Geeignete magensaftresistente Stoffe für den erfindungsgemässen Zweck sind beispielsweise Celluloseacetatphthalat, Hydroxypropyl-methylcellulosephthalat, Polyvinylacetatphthalat, Methacrylsäure und Methacrylsäureester.

Diese magensaftresistenten Stoffe können mit oder ohne Weichmacher, wie acetylierte Glycerid oder Diäthylphthalat nach an sich bekannten Methoden, von denen einige nachstehend beschrieben sind, aufgebracht werden.

Der Prozentsatz an magensaftresistentem Ueberzug liegt im allgemeinen zwischen etwa 1 und etwa 10 Gew.-% oder mehr, vorzugsweise zwischen etwa 2 und etwa 8 Gew.-%, basierend auf dem Tabletten-oder Kapselgesamtgewicht. Beispiele geeigneter magensafttresistenter Formulierungen sind nachstehend angeführt.

| Inhaltsstoffe | Gew.-% |
|---|---|
| Lösung A: | |
| Hydroxypropyl-methylcellulosephthalat (HPMCP) | 5.0 |
| Triacetin | 0.5 |
| Methylenchlorid | 47.25 |
| Denaturiertes Alkohol | 47.25 |
| Lösung B: | |
| HPMCP | 10.0 |
| Titandioxid | 0.2 |
| Dimethylpolysiloxan | 0.05 |
| Aceton | 44.875 |
| Denaturierter Alkohol | 44.875 |
| Lösung C: | |
| Cellulosacetatphthalat (CAP) | 8.5 |
| Diäthylphthalat | 1.5 |
| Titandioxid | 0.2 |
| Aceton | 44.9 |
| Denaturierter Alkohol | 44.9 |
| Lösung D: | |
| Polyvinylacetatphthalat | 5.0 |
| Acetylierte Glyceride | 0.8 |
| Methylechlorid | 47.1 |
| Denaturierter Alkohol | 47.1 |

Lösung E:

| | |
|---|---|
| Methacrylsäure oder Methacrylsäureester (Eudragit S oder L, Röhm Pharma, GMBH, Wetterstadt, Bundesrepublik Deutschland) | 8.0 |
| Aceton | 46.0 |
| wasserfreier Alkohol | 46.0 |
| Weichmacher | q.s. |

Die erfindungsgemässen Präparate können zusätzlich die üblichen Mengen von Zusätzen oder Hilfsstoffen konventioneller Art für pharmazeutische Präparate enthalten. Beispiele dafür sind Verdickungsmittel wie Kieselsäure (beispielsweise Aerosil-Produkte), Bentonite, kolloidaler Ton, Carboxymethylcellulose, modifizierte Montmorillonite wie Alkylammoniumsalze von Montmorilloniten (beispielsweise Bentone), organische Verdickungsmittel und Strukturbildner wie gesättigte höhere Fettsäuren und Alkohole mit 12-20 C-Atomen (beispielsweise Stearin-und Palmitinsäure oder Stearyl-oder Cetylalkohol), Wachse, Spermaceti, Monoglyceride gesättigter oder ungesättiger höherer Fettsäuren wie Stearinsäure, Palmitinsäure oder Oelsäure, Geliermittel wie Aluminiumstearat, Dispersionsmittel wie anionische, nicht-ionische oder kationische oberflächenaktive Stoffe, Emulgatoren wie Lecithin usw.

Die Präparate können auch pharmazeutisch anwendbare Hilfsstoffe wie Bindemittel und Schmiermittel zur Tablettierung, Stabilisatoren, Antioxidantien, Geschmackstoffe, Konservierungsmittel, Farbstoffe und Puffer enthalten.

Die verbesserte Resorption oral verabreichbarer erfindungsgemässer Präparate wurde mit in vivo Tests ermittelt.

Eine erste in vivo-Testanordnung war wie folgt: Als Versuchsobjekte wurden Menschen und Ratten eingesetzt, die ein $\beta$-Lactam-Antibiotikum sowohl oral als auch enteral erhielten. Für die orale Verabreichung wurden die Antibiotika in destilliertem Wasser oder in einem Träger wie Witepsol H15 allein oder zusammen mit CAPMUL MCM 90 und einem Resorptionsverstärker wie Natriumchenodeoxycholat (NaCDC) oder Chenodeoxycholsäure oder Natriumdeoxycholat (NaDC) oder Deoxycholsäure verwendet. In de gleichen Weise wurden die Antibiotika für die enterale Verabreichung hergerichtet und duodenal verabreicht.

Plasmaspiegel der Antibiotika wurden im Blut aus der Armvene des Menschen und der Schwanzvene der Ratte nach Zentrifugieren bestimmt. Die Antibiotika-enthaltenden Blutproben von Ratten wurden durch Bioassay auf einer Nunc-Platte analysiert; E. coli 1346, über Nacht auf einer Antibiotika--Agar Nr. 1-Kultur kultiviert wurde mit Kochsalzlösung gewaschen, wobei eine Suspension mit 90% Durchlässigkeit auf einem Bausch und Lomb-Spectronic 20 (650 nm) hergestellt wurde. 16 ml der erhaltenen Suspension wurden zu 600 ml geschmolzenem Antibiotika-Agar Nr. 1 gegeben, 200 ml des beimpften Agars wurden auf jede Nunc-Platte (243 $\times$ 243 $\times$18 mm) gegeben. Die Agarplatten wurden ausgestanzt, so dass 20 ml Proben in jede Höhlung gegeben werden konnten. Bei Ceftriaxon-Proben wurde Acetonitril zur Deproteinisierung der Probe vor dem Test verwendet.

Die das Antibiotikum enthaltenden Blutproben von Menschen wurden wie von I.H. Patel et al., "Multiple Intravenous Dose Pharmacocinetics of Ceftriaxone in Man", Chemotherapy 27 (Suppl. 1), 47-56 (1981), Seite 49, beschrieben, analysiert.

Die Resultate (Blutspiegel des Antibiotikums in Mikrogram pro ml (mg/ml) und die prozentuale Bioverfügbarkeit bei Menschen und Ratten nach einer bestimmten Dosis des Antibiotikums in Wasser oder mit dem Resorptionsverstärker sind in Tabelle 1 angegeben.

Die Tabelle zeigt, dass signifikant höhere Spiegel von Ceftiaxon, Cefamandol, Carumonam, Amdinocillin, Moxalactam und Thienamycin erhalten wurden, wenn diese Antibiotika mit Natriumchenodeoxycholat anstelle von Wasser verabreicht wurden. Der Spiegel von Ceftriaxon wird auch durch Natriumdeoxycholat, wie gezeigt, erhöht. Die Tabelle zeigt auch, dass die Resorption von Ceftriaxon durch CAPMUL MCM 90 synergistisch verstärkt wird.

Tabelle 1

Antibiotika-Blutspiegel

| Nr. | Formulierung | | Spezies | % Bioverfügbarkeit | Cmax (µg/ml) |
|---|---|---|---|---|---|
| 1)* | Ceftriaxon | (1.5 g) | Mensch | > 1 1 | 1.55 |
| | Witepsol H15 | (2.95g) | | | |
| 2) | Ceftriaxon | (1.5g) | Mensch | etwa 5 | 10.7 |
| | NaCDC | (0.25g) | | | |
| | Witepsol H15 | (2.95g) | | | |
| 3) | Ceftriaxon | (1.0g) | Mensch | etwa 10 | 12.8 |
| | NaCDC | (0.25g) | | | |
| | CAPMUL MCM 90 | (0.75g) | | | |
| | Witepsol H15 | (1.8g) | | | |
| 4)* | Ceftriaxon | (6 mg) | Ratte | 3.4 | 2.0 |
| | Wasser | (0.5 ml) | | | |
| 5) | Ceftriaxon | (6 mg) | Ratte | 28.1 | 23.5 |
| | NaCDC | (2.5 mg) | | | |
| | Wasser | (0.5 ml) | | | |
| 6)* | Ceftriaxon | (6 mg) | Ratte | 4.5 | 2.5 |
| | Witepsol H15 | (14 mg) | | | |
| 7) | Ceftriaxon | (6 mg) | Ratte | 28.0 | 27.0 |
| | NaCDC | (2.5 mg) | | | |
| | WitepsolH15 | (11.5 mg) | | | |

Tabelle 1

Antibiotika-Blutspiegel

| Nr. | Formulierung | | Spezies | % Bioverfügbarkeit | Cmax (µg/ml) |
|---|---|---|---|---|---|
| 8) | Ceftriaxon | (6 mg) | Ratte | 4.5 | 5.6 |
| | CAPMUL MCM 90 | (10µl) | | | |
| | Wasser | (0.5 ml) | | | |
| 9) | Ceftriaxon | (6 mg) | Ratte | 43.0 | 46.0 |
| | NaCDC | (2.5 mg) | | | |
| | CAPMUL MCM 90 | (10 µl) | | | |
| | Wasser | (0.5 ml) | | | |
| 10) | Ceftriaxon | (6 mg) | Ratte | 23.7 | 22.7 |
| | NaDC | (2.5mg) | | | |
| | Wasser | (0.5 ml) | | | |
| 11)* | Cefamandol | (5 mg) | Ratte | 7.0 | 1.3 |
| | Water | (0.5 ml) | | | |
| 2) | Cefamandol | (5 mg) | Ratte | 39.5 | 11.3 |
| | NaCDC | (2.5 mg) | | | |
| | Wasser | (0.5 ml) | | | |

Tabelle 1

Antibiotika-Blutspiegel

| Nr. | Formulierung | | Spezies | % Bioverfügbarkeit | Cmax (µg/ml) |
|---|---|---|---|---|---|
| 13)* | Carumonam | (5 mg) | Ratte | 0.0 | 0.0 |
| | Wasser | (0.5 ml) | | | |
| 14) | Carumonam | (5 mg) | Ratte | 15.9 | 2.0 |
| | NaCDC | (2.5 mg) | | | |
| | Wasser | (0.5 ml) | | | |
| 15)* | Amdinocillin | (5 mg) | Ratte | 6.0 | 0.7 |
| | Wasser | (0.5 ml) | | | |
| 16) | Amdinocillin | (5 mg) | Ratte | 37.0 | 6.8 |
| | NaCDC | (2.5 mg) | | | |
| | Wasser | (0.5 ml) | | | |
| 17)* | Moxalactam | (5 mg) | Ratte | 0.0 | 0.0 |
| | Wasser | (0.5 ml) | | | |
| 18) | Moxalactam | (5 mg) | Ratte | 16.2 | 3.7 |
| | NaCDC | (2.5 mg) | | | |
| | Wasser | (0.5 ml) | | | |
| 19)* | Thienamycin | (5 mg) | Ratte | 0.0 | 0.0 |
| | Wasser | (0.5 ml) | | | |

Tabelle 1

Antibiotika-Blutspiegel

| Nr. | Formulierung | | Spezies | % Bioverfübbarkeit | Cmax (µg/ml) |
|---|---|---|---|---|---|
| 20) | Thienamycin<br>NaCDC<br>Wasser | (5 mg)<br>(10.0 mg)<br>(0.5 ml) | Ratte | 100.0 | 52.0 |

* Kontrolle oder Vergleichspräparat

Zusätzlich wurde die Wirkungsverstärkung von oral verabreichtem Insulin durch die erfindungsgemässen Präparate in einem in vivo Test untersucht.

In dieser Versuchsanordnung wurden Ratten über Nacht fasten gelassen, anästhesiert und der Wirkstoff durch Injektion in das Duodenum verabreicht. Blutproben aus dem Schwanz wurden zu verschiedenen Zeiten entnommen und auf chemisch vorbehandeltem Papier (Chemistrip bg. Boehringer Mannheim Diagnostics) mittels eines Accucheck bg Blutglukosemonitors (Biodynamics, Baltimore) ausgewertet.

Die folgenden Präparate wurden so verabreicht:

1) Wasser 0.5 ml
Insulin 0 Einheiten
NaCDC 0 mg

2) Wasser 0.5 ml
Insulin 10 Einheiten
NaCDC 0 mg

3) Wasser 0.5 ml
Insulin 10 Einheiten
NaCDC 2.5 mg

4) Wasser 0.5 ml
Insulin 20 Einheiiten
NaCDC 0 mg

5) Wasser 0.5 ml
Insulin 20 Einheiten
NaCDC 2.5 mg

Die mit diesen Präparaten erhaltenen Blutglukosespiegel in mg/dl (mg%) bei Ratten sind in der nachstehenden Tabelle 2 angegeben.

Daraus wird ersichtlich, dass die Anwesenheit von Natriumchenodeoxycholat (NaCDC) in den Präparaten in einer signifikanten zeitweisen Verminderung des Blutzuckers resultierte, die die durch NaCDC verursachte verstärkte Insulinaufnahme anzeigt. Das anfängliche Ansteigen des Blutglukosespiegels ist eine vorherzusehende Reaktion auf die Anästhesie und Chirugie.

## Tabelle 2
## Insulineffekte auf dem Blutglukosespiegel mit und ohne Verstärker (Ratten)

| | Blutglukosespiegel, mg/dl | | | | |
|---|---|---|---|---|---|
| | Formulierung | | | | |
| Zeit (min.) | Nr. 1 ($H_2O$) | Nr. 2 Insulin 10 | Nr. 3 Insulin 10 + NaCDC | Nr. 4 Insulin 20 | Nr. 5 Insulin 20 + NaCDC |
| bei Anästhesie | 67.5 | 74.0 | 74.0 | 82.5 | 73.5 |
| bei Injektion | 99.0 | 95.3 | 106.0 | 117.0 | 115.0 |
| 15 | 83.2 | 83.0 | 84.6 | 94.5 | 73.0 |
| 30 | 77.5 | 80.5 | 52.0 | 95.0 | 47.0 |
| 60 | 73.0 | 81.8 | 57.0 | 90.0 | 38.5 |
| 90 | 81.0 | 72.5 | 68.0 | 80.3 | 43.8 |
| 120 | 71.0 | 65.5 | 64.6 | 82.5 | 43.5 |
| 150 | 69.5 | 73.0 | 76.0 | 92.8 | 67.8 |

Bei den vorstehend beschriebenen Versuchen wurden Lösungen des Wirkstoffs in destilliertem Wasser bei Raumtemperatur hergestellt und sofern ein Resorptionsverstärker anwesend war, dieser in feinverteilter Form langsam eingeführt, wobei eine feinteilige Suspension erhalten wurde.

Nachstehend wird ein verfahren beschrieben, dass zur Herstellung der erfindungsgemässen oral verabreichten Präparate in Form von Weichgelatinekapseln verwendet werden kann.

10'000 g Ceftriaxon und 2'500 g Natriumdeoxycholat wurden bei Raumtemperatur in einem Walzenmischer gemischt. In einem anderen Behälter wurden 17'500 g Witepsol H15 durch Erwärmen auf 40-50°C geschmolzen. Das Gemisch von Ceftriaxon und Natriumdeoxycholat wurde langsam in die Witepsolschmelze gegeben und zu einer homogenen Dispersion vermischt. Danach wurden 7'500 g Capmul MCM 90 zugesetzt und das Gemisch zur Homogenität gemischt. Die erhaltene Dispersion wurde entgast und dann auf 40°C gebracht. Die Dispersion wurde auf einer Standard-Weichgelatineverkapselungsmaschine in Weichgelatinekapseln abgefüllt. Nach Trocknen wurden die Kapseln mit einem magensaftresistenten Ueberzug in üblicher Weise versehen.

Nachstehend sind Formulierungen für Präparate mit verschiedenen Antibiotika und Insulin aufgeführt.

## Beispiel 1

| Inhaltsstoffe | mg / Dosiseinheit 25 mg | 500 mg | 1000 mg |
|---|---|---|---|
| Wirkstoff, z.B. Ceftriaxon, Cefamandol, Cefazolin, Cefoxitin, Carumonam, Aztreonam, Amdinocillin, Moxalactam, Cefotaxim, Piperacillin, Mezclocillin, (6R,7R)-7-[(Z)-2-(2-Amino-4-thiazolyl)-2-(methoxyimino)acetamido]-3-[(5-methyl-2H-1,2,3,4-tetrazol-2-yl)methyl]-8-oxo-5-thia-1-azabicyclo[4.2.0]oct-2-en-2-carbonsäure-Natriumsalz Cefmenoxim, Cefoperazon, Cefsulodin oder Thienamycin | 25 mg | 500 mg | 1000 mg |
| Natriumchenodeoxycholat | 250 mg | 250 mg | 250 mg |
| Witepsol H15, q.s. | 1000 mg | 2000 mg | 3000 mg |
| Total | 1000 mg | 2000 mg | 3000 mg |

Die folgenden Beispiele illustrieren orale Dosierungsformen, in denen zwei Absorptionsverstärker in Kombination angewandt wurden. Capmul MCM 90 ist ein Gemisch von Glyceriden gesättigter Fettsäuren, die etwa 90% Monoglyceride im $C_8$-$C_{10}$-Bereich enthalten.

## Beispiel 2

| Inhaltsstoffe | mg / Dosiseinheit 25 mg | 500 mg | 1000 mg |
|---|---|---|---|
| Wirkstoff, z.B. Ceftriaxon Cefamandol, Cefazolin, Cefoxitin, Carumonam, Aztreonam, Amdinocillin, Moxalactam, Cefotaxim, Piperacillin, Mezclocillin, (6R,7R)-7-[(Z)-2-(2-Amino-4-thiazolyl)-2-(methoxyimino)acetamido]-3-[(5-methyl-2H-1,2,3,4-tetrazol-2-yl)methyl]-8-oxo-5-thia-1-azabicyclo[4.2.0]oct-2-en-2-carbonsäure-Natriumsalz Cefmenoxim, Cefoperazon, Cefsulodin oder Thienamycin | 25 mg | 500 mg | 1000 mg |
| Natriumchenodeoxycholat | 250 mg | 250 mg | 250 mg |
| Capmul MCM 90 | 750 mg | 750 mg | 750 mg |
| Witepsol H15, q.s. | 2000 mg | 3000 mg | 4000 mg |
| Total | 2000 mg | 3000 mg | 4000 mg |

**Ansprüche**

1. Oral verabreichbares pharmazeutisches Präparat, enthaltend (a) eine therapeutisch wirksame Substanz, deren Bioverfügbarkeit in Gegenwart eines Resorptionsverstärkers verbessert wird, eine wirksame Menge von (b) (i) Chenodeoxycholsäure, Deoxycholsäure oder pharmazeutisch verwendbare Salze davon, gegebenenfalls in Kombination mit (ii) einem Synergisten für die Komponente (b) (i), und (c) einen pharmazeutisch anwendbaren Träger für diese Inhaltsstoffe.

2. Präparat nach Anspruch 1 zur Herstellung eines Präparats in fester Form.

3. Präparat nach Anspruch 1, wobei die therapeutisch wirksame Substanz ein Antibiotikum, ein Retinoid, ein Peptid, ein Polypeptid, ein Protein, ein Vitamin oder ein ernährungswichtiges Mineral ist.

4. Präparat nach Anspruch 3, wobei das Antibiotikum ein $\beta$-Lactam-Antibiotikum ist.

5. Präparat nach Anspruch 4, wobei das $\beta$-Lactam-Antibiotikum Ceftriaxon, Cefamandol, Cefazolin, Cefoxitin, Carumonam, Aztreonam, Amdinocillin, Moxalactam, Cefotaxim, Piperacillin, Mezlocillin, Cefmenoxim, Cefoperazon, Cefulodin oder Thienamycin ist.

6. Präparat nach Anspruch 3, wobei das Protein Insulin ist.

7. Präparat nach den Ansprüchen 1-6, wobei die Komponente (b)(i) Natriumchenodeoxycholat oder Natriumdeoxycholat ist.

8. Präparat nach den Ansprüchen 1-7, wobei die Komponente (b)(ii) ein Fettsäureester ist.

9. Präparat nach Anspruch 8, wobei der Fettsäureester ein Mono-, Di-oder Triglycerid einer Fettsäure mittlerer Kettenlänge oder ein Gemisch von mehreren solcher Glyceride ist.

10. Präparat nach Anspruch 1, wobei der Resorptionsverstärker ein Gemisch von Natriumdeoxycholat und Glyceriden von $C_8$-$C_{12}$-Fettsäuren ist.

11. Präparat nach Anspruch 4, enthaltend etwa 25-2500 mg β-Lactam-Antibiotikum und etwa 5-1000 mg Resorptionsverstärker pro Dosiseinheit.

12. Präparat nach Anspruch 6, enthaltend etwa 0,1-20'000 Einheiten Insulin und etwa 5-1000 mg Resorptionsverstärker pro Dosiseinheit.

13. Präparat nach Anspruch 1 in Form einer Weichgelatinekapsel mit magensaftresistentem Ueberzug.

14. Verwendung von (i) Chenodeoxycholsäure, Deoxycholsäure, oder pharmazeutisch verwendbaren Salzen davon, gegebenenfalls in Kombination mit (ii) einem Synergisten für die Komponente (i) als Resorptionsverstärker zur Herstellung von oral verabreichbaren pharmazeutischen Präparaten.

15. Verwendung nach Anspruch 14 zur Herstellung von pharmazeutischen Präparaten, die als therapeutisch wirksame Substanz ein Antibiotikum, ein Retinoid, ein Peptid, ein Polypeptid, ein Protein, ein Vitamin oder ein ernährungswichtiges Mineral enthalten.

16. Verwendung nach Anspruch 14 zur Herstellung von pharmazeutischen Präparaten, die als therapeutisch wirksame Substanz ein β-Lactam-Antibiotikum enthalten.

17. Verwendung nach Anspruch 14 zur Herstellung von pharmazeutischen Präparaten, die als therapeutisch wirksame Substanz Insulin enthalten.

Patentansprüche für folgende Vertragsstaaten : GR; AT; ES.

1. Verfahren zur Herstellung oral verabreichbarer pharmazeutischer Präparate mit verbesserter gastrointestinaler Resorption des Wirkstoffs, dadurch gekennzeichnet, dass man (a) eine therapeutisch wirksame Substanz, deren Bioverfügbarkeit in Gegenwart eines Resorptionsverstärkers verbessert wird, eine wirksame Menge von (b) (i) Chenodeoxycholsäure, Deoxycholsäure oder pharmazeutisch verwendbare Salze davon, gegebenenfalls in Kombination mit (ii) einem Synergisten für die Komponente (b) (i), und (c) einen pharmazeutisch anwendbaren Träger für diese Inhaltsstoffe, in eine oral verabreichbare Anwendungsform bringt.

2. Verfahren nach Anspruch 1 zur Herstellung eines Präparats in fester Form.

3. Verfahren nach Anspruch 1, wobei die therapeutisch wirksame Substanz ein Antibiotikum, ein Retinoid, ein Peptid, ein Polypeptid, ein Protein, ein Vitamin oder ein ernährungswichtiges Mineral ist.

4. Verfahren nach Anspruch 3, wobei das Antibiotikum ein β-Lactam-Antibiotikum ist.

5. Verfahren nach Anspruch 4, wobei das β-Lactum-Antibiotikum Ceftriaxon, Cefamandol, Cefazolin, Cefoxitin, Carumonam, Aztreonam, Amdinocillin, Moxalactam, Cefotaxim, Piperacillin, Mezlocillin, Cefmenoxim, Cefoperazon, Cefulodin oder Thienamycin, ist.

6. Verfahren nach Anspruch 3, wobei das Protein Insulin ist.

7. Verfahren nach den Ansprüchen 1-6, wobei die Komponente (b))i) Natriumchenodeoxycholat oder Natriumdeoxycholat ist.

8. Verfahren nach den Ansprüchen 1-7, wobei die Komponente (b)(ii) ein Fettsäureester ist.

9. Verfahrwen nach Anspruch 8, wobei der Fettsäureester ein Mono-, Di-oder Triglycerid einer Fettsäure mittlerer Kettenlänge oder ein Gemisch von mehreren solcher Glyceride ist.

10. Verfahren nach Anspruch 1, wobei der Resorptionsverstärker ein Gemisch von Natriumdeoxycholat und Glyceriden von $C_8$-$C_{12}$-Fettsäuren ist.

11. Verfahren nach Anspruch 4, wobei man etwa 25-2500 mg β-Lactam-Antibiotikum und etwa 5-1000 mg Resorptionsverstärker pro Dosiseinheit verwendet.

12. Verfahren nach Anspruch 6, wobei man etwa 0,1-20'000 Einheiten Insulin und etwa 5-1000 mg Resorptionsverstärker pro Dosiseinheit verwendet.

13. Verfahren nach Anspruch 1, wobei man das Präparat in die Form einer Weichgelatinekapsel mit magensaftresistentem Ueberzug bringt.